# EUROPEAN PATENT APPLICATION

(11) **EP 4 620 518 A1**
(43) Date of publication of application: **24.09.2025**
(21) Application number: 23891910.4
(22) Date of filing: 08.11.2023
(51) Int. Cl.: A61N 1/40, A61N 1/06, A61N 1/32, A61N 1/36

(54) **COUPLING STRUCTURE OF CARTRIDGE FOR SKIN TREATMENT**

(30) Priority: 17.11.2022 KR 20220154149
(71) Applicant: CLASSYS INC., Seoul 06220 (KR)
(72) Inventor: KO, Min Soo, Seoul 01632 (KR)
(74) Representative: Wörz, Volker Alfred
(86) International application number: PCT/KR2023/017868
(87) International publication number: WO 2024/106843

(57) **Abstract**

The present invention relates to a coupling structure of a cartridge for skin treatment. It is possible to simply couple and separate a cartridge for skin treatment, having equipped therein an operation part which touches and treats a patient's skin, to and from an object to which the cartridge is to be coupled, by means of a ball plunger structure. Thus, convenience is greatly improved when replacing, cleaning, or disinfecting the cartridge for skin treatment, and the electrical connection structure for electrically connecting the cartridge for skin treatment to the object to which the cartridge is to be coupled is simplified, and thereby manufacturing costs can be reduced.

## Description

### [Technical Field]

The present disclosure relates to a coupling structure of a cartridge for skin treatment, and more particularly, to a coupling structure of a cartridge for skin treatment that can ensure convenience when replacing an electrode cartridge for skin treatment that is periodically replaced.

### [Background Art]

In recent years, interest in skin care has been increasing day by day, and various skin treatment devices have been developed.

Skin treatment devices have been proposed in various forms, such as a skin treatment device using ultrasonic waves a skin treatment device using a laser, and a skin treatment device using a radio frequency (RF) electrode.

A skin treatment device using ultrasonic waves includes an ultrasound treatment handpiece that comes into contact with the skin of a subject and emits ultrasonic waves into the skin, and a main body that controls the operation of the ultrasound treatment handpiece.

A skin treatment device using ultrasonic waves generates thermal lesions induced by high-intensity focused ultrasonic waves in skin tissues at a certain depth from the skin surface using an ultrasound treatment handpiece, and is mainly used for procedures that deliberately damage specific skin tissue or melt and break down fat tissue.

Further, skin treatment devices using a laser are used for various purposes such as preventing hair loss, promoting hair growth, skin peeling, skin regeneration, whitening, removal of wrinkles or spots, and removal of melasma, and perform treatment in a manner that damages skin tissues by emitting a laser onto the skin in the form of dots of a predetermined size, that is, applying heat only to the treatment object area using a laser.

Recently, skin treatment devices using a radio frequency (RF) electrode are used in various ways such as skin remodeling/resurfacing, wrinkle removal, and the treatment of sebaceous glands, fatty tissues around hair follicles, and spider veins by causing contraction or wound-healing response in the collagen within the lower dermis.

In skin treatment devices, a procedure is performed by an operator holding a handpiece to which a treatment cartridge is detachably coupled, bringing the treatment cartridge into contact with the skin, and then operating the treatment cartridge.

Further, treatment cartridges have a preset number of uses, and once they have been used the preset number of times, they must be replaced from the handpiece.

Procedure cartridges such as an ultrasound procedure cartridge, a laser procedure cartridge, and an RF electrode procedure cartridge are electrically connected when coupled to a handpiece, and their operation is controlled by receiving electric power from a main control unit.

Procedure cartridges in the related art had a problem in that they were structurally complex and costly to manufacture because they were separately provided with a locking structure for maintaining engagement with a handpiece when coupled to the handpiece and an electrical contact structure for electrical connection to a main control unit.

Further, handpieces have a problem that when they have a structure that is electrically connected to a procedure cartridge through a wire upon coupling with the procedure cartridge, considerable limitations and inconvenience occur in coupling and decoupling of a procedure cartridge and in cleaning.

In particular, in the case of an RF cartridge that performs a procedure on the skin while rotating with an RF electrode in contact the skin, there is a problem in that the electrical connection structure is complicated and that significant inconvenience occurs when coupling to and decoupling from a handpiece during replacement.

A prior patent related to the present disclosure is Korean Patent No. 2206623, titled "Needle Tip Mounted on Skin Caring Device and Skin Caring Device" (registered on January 18, 2021).

### [Disclosure]

### [Technical Problem]

An objective of the present disclosure is to provide a coupling structure of a cartridge for skin treatment, which can ensure convenience when replacing an electrode cartridge for skin treatment that is periodically replaced, and can simplify an electrical connection structure.

### [Technical Solution]

In order to achieve the objectives of the present disclosure described above, a coupling structure of a cartridge for skin treatment according to the present disclosure includes: a cartridge for skin treatment having a skin treatment operation unit, which is configured to stimulate the skin of a patient, and configured to come into contact with the skin of a patient; ball plunger assemblies protruding from both sides of the cartridge for skin treatment; and a cartridge coupling target having a cartridge coupling portion in which a portion of the cartridge for skin treatment is inserted and coupled, wherein ball seats in which the ball plunger assemblies are inserted are formed on inner surfaces of the cartridge coupling portion.

In the present disclosure, the cartridge coupling object may include a handpiece body configured to be held and used by an operator and to electrically connect the skin treatment operation unit to a control body, and the ball plungers and the ball seats may have electrical terminal structures electrically connected to each other, thereby electrically connecting the skin treatment operation unit and the control body.

In the present disclosure, the ball plunger assemblies may include a bottom terminal in which a spring is seated on a bottom of a spring insertion groove, in which the spring is inserted, and that is electrically connected to the skin treatment operation unit through a wire.

In the present disclosure, the cartridge for skin treatment may have ring-shaped contact protrusions surrounding the ball plunger assemblies on both sides.

In the present disclosure, the cartridge for skin treatment may include a cylindrical cartridge body having the ball plunger assemblies on both sides, and may be rotated about the ball plunger assemblies.

In the present disclosure, the skin treatment operation unit may include an RF electrode unit positioned on an outer surface of the cartridge body and configured to apply an RF signal in contact with a skin.

In the present disclosure, ring-shaped contact protrusions surrounding the ball plunger assemblies in contact with the inner surfaces of the cartridge coupling portion may protrude from both sides of the cartridge body.

In the present disclosure, cartridge stoppers configured to prevent the cartridge for skin treatment from being pushed into the cartridge coupling portion in contact with a skin by engaging with the contact protrusions may protrude from the inner surfaces of the cartridge coupling portion.

In the present disclosure, the cartridge stoppers may have ball bearing portions, which roll in contact with the contact protrusions, on surfaces facing the contact protrusions.

An embodiment of the coupling structure of a cartridge for skin treatment according to the present disclosure may further include a cartridge separator positioned to be movable forward and backward in a coupling direction of the cartridge for skin treatment within the cartridge coupling portion, and configured to separate the cartridge for skin treatment from the cartridge coupling portion by pushing the cartridge for skin treatment.

In the present disclosure, the cartridge separator may include: a pusher positioned to be movable forward and backward inside the cartridge coupling portion; and a linear actuator positioned at the cartridge coupling object and configured to move the pusher forward and backward.

In the present disclosure, the cartridge separator may further include an operation switch unit positioned at the cartridge coupling object and configured to be operated by an operator to control operation of the linear actuator.

### [Advantageous Effects]

Since it is possible to simply couple and separate a cartridge for skin treatment to a cartridge coupling object using a ball plunger structure, the present disclosure has the effect that it is possible to significantly improve the convenience of replacement, and cleaning or disinfection of the cartridge for skin treatment.

Further, an electrical connection structure that electrically connects the cartridge for skin treatment to the cartridge coupling object is simplified by the ball plunger structure that couples the cartridge for skin treatment to the cartridge coupling object, so the present disclosure has the effect that the manufacturing costs are reduced.

### [Description of Drawings]

FIG. 1 is a perspective view showing an embodiment of a coupling structure of a cartridge for skin treatment according to the present disclosure.
FIG. 2 is an exploded perspective view showing an embodiment of a coupling structure of a cartridge for skin treatment according to the present disclosure.
FIG. 3 is a partial cross-sectional view showing an embodiment of a coupling structure of a cartridge for skin treatment according to the present disclosure.
FIG. 4 is a plan view showing another embodiment of a coupling structure of a cartridge for skin treatment according to the present disclosure.

***Description of main reference numerals in drawings**

| | | | |
|---|---|---|---|
| 100 : | cartridge for skin treatment | 100a : | contact protrusion |
| 110 : | cartridge body | 120 : | RF electrode unit |
| 200 : | ball plunger assembly | 210 : | ball |
| 220 : | spring | 230 : | bottom terminal |
| 300 : | cartridge coupling object | 300a : | handpiece body |
| 310 : | cartridge coupling portion | 320 : | ball seat |
| 321 : | ball insertion terminal | 330 : | cartridge stopper |
| 340 : | ball bearing portion | 400 : | cartridge separator |
| 410 : | pusher | 420 : | linear actuator |
| 430 : | operation switch unit | 431 : | forward switch |
| 432 : | reverse switch | | |

### [Best Mode]

Hereafter, the present disclosure is described in more detail.

Exemplary embodiments of the present disclosure are described hereafter in detail with reference to the accompanying drawings. Before describing the present disclosure, it should be noted that the terms or terminologies used herein and claims should not be construed as common meanings or the meanings in dictionaries. Therefore, the configurations described in the embodiments and drawings of the present disclosure are merely exemplary embodiments but do not represent all of the technical spirit of the present invention. Thus, it should be understood that the present disclosure should be construed as including all the changes, equivalents, and substitutions included in the spirit and scope of the present disclosure at the time of filing this application.

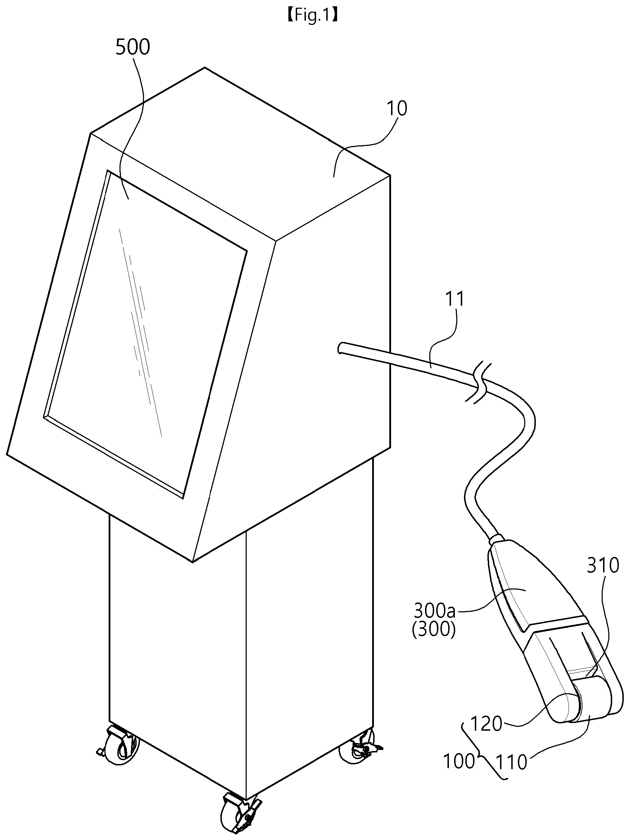

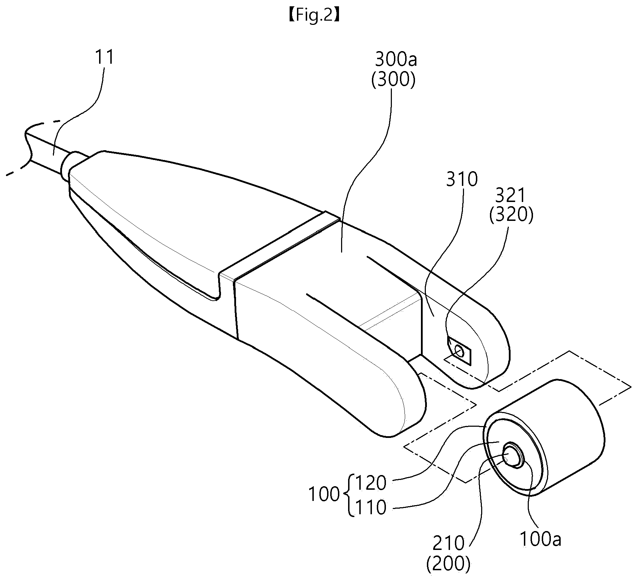

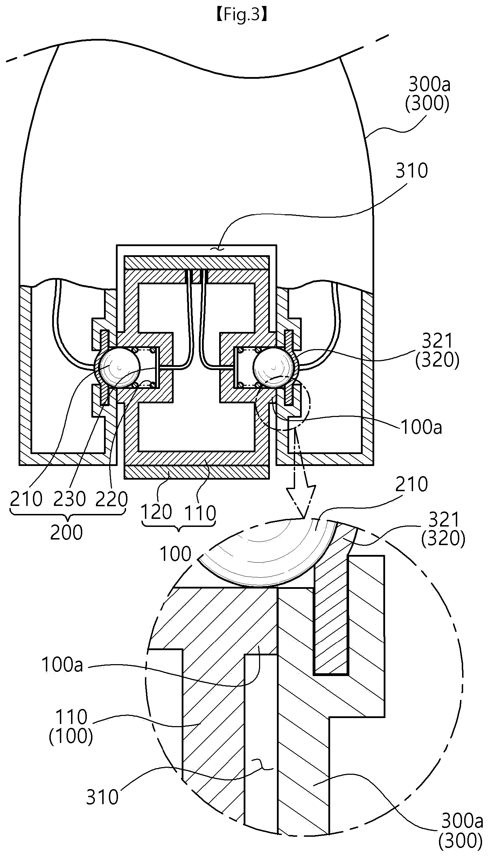

FIG. 1 is a perspective view showing an embodiment of a coupling structure of a cartridge for skin treatment according to the present disclosure, FIG. 2 is an exploded perspective view showing an embodiment of a coupling structure of a cartridge for skin treatment according to the present disclosure, and FIG. 3 is a partial cross-sectional view showing an embodiment of a coupling structure of a cartridge for skin treatment according to the present disclosure.

An embodiment of a coupling structure of a cartridge for skin treatment according to the present disclosure is described hereafter in detail with reference to FIGS. 1 to 3.

An embodiment of the coupling structure of a cartridge for skin treatment according to the present disclosure includes a cartridge 100 for skin treatment that comes into contact with the patient's skin and has a skin treatment operation unit that stimulates the patient's skin.

Further, an embodiment of the coupling structure of a cartridge for skin treatment according to the present disclosure further includes a cartridge coupling object 300 having a cartridge coupling portion 310 into which a portion of the cartridge 100 for skin treatment is inserted and coupled.

The cartridge 100 for skin treatment has a skin treatment operation unit that operates and stimulates the skin by receiving electric power, and the skin treatment operation unit is electrically connected to the cartridge coupling object 300 when the cartridge 100 for skin treatment is coupled to the cartridge coupling object 300, and operates by receiving electric power from the cartridge coupling object 300.

The cartridge 100 for skin treatment may be implemented in a modified form as a known skin treatment cartridge that treats the skin, such as an ultrasound treatment cartridge, a laser treatment cartridge, or an RF treatment cartridge including an RF electrode that come into contact with the skin and stimulate the skin for treatment.

That is, the skin treatment operation unit, as an example, includes at least one of an ultrasound transducer that generates ultrasonic waves, a laser generating unit that generates a therapeutic laser, and an RF electrode unit 120 that is mounted on the outer surface of a cartridge housing and generates an RF signal.

Meanwhile, the cartridge coupling object 300, as an example, is a handpiece body 300a that is detachably coupled with the cartridge 100 for skin treatment, that an operator holds and uses with their hand, and that electrically connects the skin treatment operation unit with a control body 10.

The handpiece body 300a is connected to the control body 10 through a connection cable 11, and the control body 10 includes a controller that is electrically connected to the cartridge 100 for skin treatment through the handpiece body 300a and controls the operation of the skin treatment operation unit of the cartridge 100 for skin treatment.

The control body 10 is well known and can be variously modified and implemented to operate and control the operation of the handpiece body 300a, which includes various known skin treatment cartridges, so it is noted that a more detailed description is omitted.

The control body 10 may include a display panel 10a that outputs the control mode of the handpiece body 300a on the screen so that the operation of each handpiece body 300a can be controlled, and it is exemplified that the display panel 10a is a touch screen panel and an operation control signal is input by touching a displayed image.

The operation control mode of each handpiece body 300a and the input of the operation control signal in the operation control mode are known in skin treatment devices, so it is noted that a more detailed description is omitted.

A ball plunger assembly 200 protrudes on each of both sides of the cartridge 100 for skin treatment, and ball seats 321 in which the ball plunger assemblies 200 are inserted are formed on the inner surfaces of the cartridge coupling portion 310.

The ball plunger assembly 200 includes a ball 210 that partially protrudes on the side of the cartridge 100 for skin treatment, and a spring 220 positioned inside the cartridge 100 for skin treatment elastically supporting the ball 210.

Since the balls 200 partially protruding from the sides of the cartridge 100 for skin treatment are elastically supported by the springs 220, when a portion of the cartridge 100 for skin treatment is coupled into the cartridge coupling portion 310 of the handpiece body 300a that is the cartridge coupling object 300, the ball plunger assemblies are inserted into the ball seats 321 positioned on the inner surfaces of the cartridge coupling portion 310.

The cartridge 100 for skin treatment can remain coupled with the handpiece body 300a because the balls 210 of the ball plunger assemblies 200 are partially inserted in the ball seats 321 positioned on the inner surfaces of the cartridge coupling portion 310, and when the cartridge 100 for skin treatment is pulled in the opposite direction of the coupling direction such that the balls 210 are pulled out of the ball seats, the cartridge 100 for skin treatment can be easily separated from the handpiece body 300a.

That is, when the cartridge 100 for skin treatment is partially inserted into the cartridge coupling portion 310 and then pushed such that the ball plunger assemblies 200 are inserted into the ball seats 321, the cartridge 100 for skin treatment is simply coupled to the handpiece body 300a and can be used skin treatment procedures, and when the lifespan ends or a malfunction occurs, it can be easily separated, which increases convenience during replacement.

Meanwhile, the ball plunger assemblies 200 and the ball seats 321 have electrical terminal structures that are electrically connected to each other, thereby electrically connecting the skin treatment operation unit of the cartridge 100 for skin treatment and the cartridge coupling object 300, that is, the control body 10 so that electrical power can be supplied to the skin treatment operation unit and its operation can be controlled.

More specifically, the ball seat 321 is a ball insertion terminal 321 having a ball insertion groove and made of an electrically conductive material, and the ball plunger assembly 200 may have a structure in which the spring member 220 is electrically connected to the skin treatment operation unit through a wire or that further includes a bottom terminal 330 in which the spring 220 is seated on the bottom of the spring insertion groove, in which the spring 220 is inserted, and is electrically connected to the skin treatment operation unit through a wire.

The ball insertion terminal 321 is electrically connected to the control body 10 through a connection cable 11 of the handpiece body 300a.

The spring 220 always remains in contact with the ball 210 while elastically supporting the ball 210, and is electrically connected to the skin treatment operation unit through a wire or the bottom terminal 330.

The bottom terminal 330 remains in stable contact with the spring 220 by the elastic force of the spring member 220, so that it can maintain stable electrical connection between the skin treatment operation unit and the spring 220.

Accordingly, when the cartridge 100 for skin treatment is coupled into the cartridge coupling portion 310 of the handpiece body 300a, and the ball plunger assemblies 200 are inserted into the ball insertion grooves of the ball insertion terminals 321, the skin treatment operation unit is electrically connected to the control body 10 through the connection cable 11 of the handpiece body 300a, whereby the operation of the skin treatment operation unit can be controlled through the control body 10.

In the cartridge 100 for skin treatment coupled to the handpiece body 300a, the structure in which the skin treatment operation unit is electrically connected to the control body 10 through the connection cable 11 is a known structure and can be variously modified and implemented, so it is noted that a more detailed description is omitted.

Meanwhile, the skin treatment operation unit includes an RF electrode unit 120 that applies an RF signal in contact with the skin, and the cartridge 100 for skin treatment is, as an example, an RF treatment cartridge in which the RF electrode unit 120 can be rotated about the ball plunger assemblies 200 during a procedure while being in contact with the skin.

In more detail, it is exemplified that the cartridge 100 for skin treatment includes a cylindrical cartridge body 110 in which the ball plunger assemblies 200 are positioned on both sides, and is rotated about the ball plunger assemblies 200.

Ring-shaped contact protrusions 100a surrounding the ball plunger assemblies 200 in contact with the inner surfaces of the cartridge coupling portion 310 protrude from both sides of the cartridge body 110.

The contact protrusions 100a are ring-shaped protrusions surrounding the ball plunger assemblies 200 and have a width less than 30% of the diameter of the balls 210, so the contact area between both sides of the cartridge body 110 and the inner surfaces of the cartridge coupling portion 310 is minimized by the ring-shaped protrusions.

Since only the contact protrusions 100a come into contact with the inner surfaces of the cartridge coupling portion 310 when the cartridge 100 for skin treatment is coupled into and separated out of the cartridge coupling portion 310, it is possible to minimize friction and the force for coupling to and separating from the handpiece body 300a.

Since the contact protrusions 100a on both sides of the cartridge body 110 are in contact with the inner surfaces of the cartridge coupling portion 310, the cartridge body 110 can be smoothly rotated about the plunger assemblies 200.

Since the ball plunger assemblies 200 are inserted in the ball seats 321, the cartridge body 110 can be rotated about the ball plunger assemblies 200, and the ball plunger assemblies 200 can be stably maintained in an electrical connection state in the ball seats 321 while the cartridge body 110 is rotated about the ball plunger assemblies 200.

An operator holds the handpiece body 300a, brings the RF electrode unit 120 of the cartridge body 110 into contact with the skin, and performs a procedure while rotating the cartridge body 110 by pushing or pulling the handpiece body 300a to apply an RF signal to the patient's skin. In this case, the cartridge body 110 is rotated about the ball plunger assemblies 200 and the ball plunger assemblies 200 are stably maintained in an electrically connected state in the ball seats 321, so the RF signal can be stably applied to the skin.

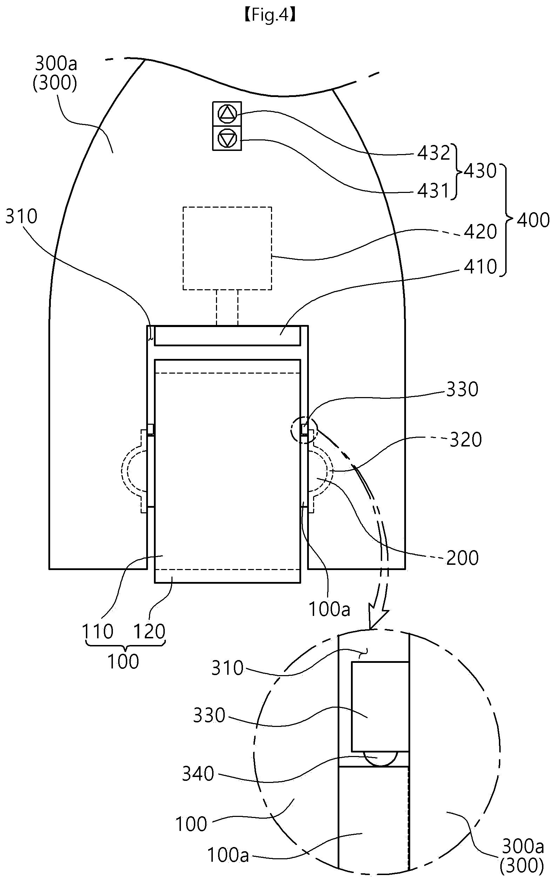

Meanwhile, FIG. 4 is a plan view showing another embodiment of a coupling structure of a cartridge for skin treatment according to the present disclosure. Referring to FIG. 4, in another embodiment of the coupling structure of a cartridge for skin treatment according to the present disclosure, a cartridge stoppers 330 configured to engage with the contact protrusions 100a protrude from the inner surfaces of the cartridge coupling portion 310.

The cartridge stoppers 330 restrict movement of the cartridge 100 for skin treatment toward the inside of the cartridge coupling portion 310 by engaging with contact protrusions 100a when the cartridge 100 for skin treatment is pushed in contact with the skin.

The cartridge stoppers 330 protrude from the inner surfaces of the cartridge coupling portion 310 toward the inside of the ball seats 321, and is positioned such that the contact protrusions 100a, which protrude from the outer surface of the cartridge 100 for skin treatment, are caught when the cartridge 100 for skin treatment that is in contact with the skin is pushed into the cartridge coupling part 310.

The cartridge 100 for skin treatment is coupled to the handpiece body 300a through the ball plunger assemblies 200, and an operator can perform a procedure by holding and pressing the handpiece body 300a with the cartridge in contact with the skin.

While an operator performs a procedure by holding the handpiece body 300a and pressing the cartridge 100 for skin treatment against the skin, the cartridge 100 for skin treatment is pushed into the cartridge coupling portion 310, whereby the ball plunger assemblies 200 may be separated from the ball seats 321.

When the ball plunger assemblies 200 are separated from the ball seats 321, a problem arises in that the power supply is cut off and the procedure is interrupted.

The cartridge stoppers 330 prevent the cartridge 100 for skin treatment, which is in contact with the skin, from moving into the cartridge coupling portion 310 when the cartridge 100 for skin treatment is pressed by an operator, by engaging the contact protrusions 100a inside the cartridge coupling portion 310, and stably keep the ball plunger portion 200 inserted in the ball seats 321 during a procedure.

Further, cartridge stoppers 330 have rolling ball bearing portions 340, which roll in contact with the contact protrusions 100a, on the surfaces facing the contact protrusions 100a, thereby allowing the cartridge body 110 to rotate smoothly.

Since the cartridge body 110 is rotated about the ball plunger assemblies 200 in contact with the skin to perform a procedure on the patient's skin, the cartridge body 110 may not be smoothly rotated due to friction caused by the contact of the contact protrusions 100a with the cartridge stoppers 330.

The ball bearing portions 340 roll in contact with the contact protrusions 100a when the cartridge body 110 is rotated in contact with the skin, thereby allowing smooth rotation of the cartridge body 110.

Accordingly, during a procedure, the cartridge body 110 is smoothly rotated in contact with the skin, and even if the cartridge body 110 is excessively pressed against the skin, the ball plunger assemblies 200 are not separated from the ball seats 321, so it is possible to stably perform a procedure and considerably improve the convenience for an operator.

Further, another embodiment of the coupling structure of a cartridge for skin treatment according to the present disclosure may further include a cartridge separator 400 that is positioned to be movable forward and backward in the coupling direction of the cartridge 100 for skin treatment within the cartridge coupling portion 310 and separates the cartridge 100 for skin treatment from the cartridge coupling portion 310 by pushing it.

The cartridge separator 400 includes a pusher 410 that is positioned to be movable forward and backward in the coupling direction of the cartridge 100 for skin treatment within the cartridge coupling portion 310, and a linear actuator 420 that is positioned inside the handpiece body 300a, which is the cartridge coupling object 300, and moves the pusher 410 forward and backward.

Further, the cartridge separator 400 may further include an operation switch unit 430 that positioned at the handpiece body 300a and that is operated an operator to control the operation of the linear actuator 420.

The operation switch unit 430 includes a forward switch 431 and a reverse switch 432, and an operator can separate the cartridge 100 for skin treatment from the handpiece body 300a by pressing the forward switch 431 to move forward the pusher 410 with the linear actuator 420 and push the cartridge 100 for skin treatment out of the cartridge coupling portion 310 with the pusher 410.

Further, when an operator presses the reverse switch 432, the linear actuator 420 moves the pusher 410 backward to the initial position.

The linear actuator 420, for example, is ball screw-type linear actuator, but may be modified in various ways using well-known elevating devices such as a rack and pinion structure that includes a rack gear and a pinion gear engaged with the rack gear and rotated by a motor and converts torque from the motor into straight movement, and a hydraulic cylinder, so it should be noted that a detailed description is omitted.

Since the cartridge 100 for skin treatment is coupled to the handpiece body 300a, an operator may have difficulty in holding the cartridge when separating it. Further, in the case of an RF treatment cartridge with an RF electrode 120 on the outer surface, if an RF signal has been applied when an operator holds the cartridge with their fingers, there is a risk of injury such as burns to the fingers.

The cartridge separator 400 pushes the cartridge 100 for skin treatment with the pusher 410 when the cartridge 100 for skin treatment is replaced, so the cartridge 100 for skin treatment can be easily and safely separated from the cartridge coupling object 300, that is, the handpiece body 300a.

According to the present disclosure, it is possible to simply couple and separate the cartridge 100 for skin treatment to and from the cartridge coupling object 300 using a ball plunger structure, so it is possible to significantly improve the convenience of replacement, and cleaning or disinfection of the cartridge 100 for skin treatment.

Further, according to the present disclosure, an electrical connection structure that electrically connects the cartridge 100 for skin treatment to the cartridge coupling object 300 is simplified by the ball plunger structure that couples the cartridge 100 for skin treatment to the cartridge coupling object 300, so the manufacturing costs can be reduced.

The present disclosure is not limited to the embodiments described above and may be modified in various ways without departing from the scope of the present disclosure and the modifications should be construed as being included in the present disclosure.

## Claims

1. A coupling structure of a cartridge for skin treatment, the coupling structure comprising:
a cartridge for skin treatment having a skin treatment operation unit, which is configured to stimulate the skin of a patient, and configured to come into contact with the skin of a patient;
ball plunger assemblies protruding from both sides of the cartridge for skin treatment; and
a cartridge coupling object having a cartridge coupling portion in which a portion of the cartridge for skin treatment is inserted and coupled,
wherein ball seats in which the ball plunger assemblies are inserted are formed on inner surfaces of the cartridge coupling portion.

2. The coupling structure of claim 1, wherein the cartridge coupling object comprises a handpiece body configured to be held and used by an operator and to electrically connect the skin treatment operation unit to a control body, and
the ball plungers and the ball seats have electrical terminal structures electrically connected to each other, thereby electrically connecting the skin treatment operation unit and the control body.

3. The coupling structure of claim 2, wherein the ball plunger assemblies comprise a bottom terminal in which a spring is seated on a bottom of a spring insertion groove, in which the spring is inserted, and that is electrically connected to the skin treatment operation unit through a wire.

4. The coupling structure of claim 1, wherein the cartridge for skin treatment has ring-shaped contact protrusions surrounding the ball plunger assemblies on both sides.

5. The coupling structure of claim 2, wherein the cartridge for skin treatment comprises a cylindrical cartridge body having the ball plunger assemblies on both sides, and is rotated about the ball plunger assemblies.

6. The coupling structure of claim 5, wherein the skin treatment operation unit comprises an RF electrode unit positioned on an outer surface of the cartridge body and configured to apply an RF signal in contact with a skin.

7. The coupling structure of claim 5, wherein ring-shaped contact protrusions surrounding the ball plunger assemblies in contact with the inner surfaces of the cartridge coupling portion protrude from both sides of the cartridge body.

8. The coupling structure of claim 4 or 7, wherein cartridge stoppers configured to prevent the cartridge for skin treatment from being pushed into the cartridge coupling portion in contact with a skin by engaging with the contact protrusions protrude from the inner surfaces of the cartridge coupling portion.

9. The coupling structure of claim 8, wherein the cartridge stoppers have ball bearing portions, which roll in contact with the contact protrusions, on surfaces facing the contact protrusions.

10. The coupling structure of claim 1, further comprising
a cartridge separator positioned to be movable forward and backward in a coupling direction of the cartridge for skin treatment within the cartridge coupling portion, and configured to separate the cartridge for skin treatment from the cartridge coupling portion by pushing the cartridge for skin treatment.

11. The coupling structure of claim 10, wherein the cartridge separator comprises:
a pusher positioned to be movable forward and backward inside the cartridge coupling portion; and
a linear actuator positioned at the cartridge coupling object and configured to move the pusher forward and backward.

12. The coupling structure of claim 11, wherein the cartridge separator further comprises an operation switch unit positioned at the cartridge coupling object and configured to be operated by an operator to control operation of the linear actuator.
